# EUROPÄISCHE PATENTSCHRIFT

(11) **EP 0 559 853 B1**
(45) Veröffentlichungstag und Bekanntmachung des Hinweises auf die Patenterteilung: **16.10.1996**
(21) Anmeldenummer: 92917643.6
(22) Anmeldetag: 13.08.1992
(51) Int. Cl.: G01N 33/68, G01N 33/74, C12Q 1/37, C12N 9/96

(54) **VERFAHREN ZUR STABILISIERUNG VON ENDOGENEN, PHYSIOLOGISCH AKTIVEN PEPTIDEN**
METHOD OF STABILIZING ENDOGENIC, PHYSIOLOGICALLY ACTIVE PEPTIDES
PROCEDE DE STABILISATION DE PEPTIDES ENDOGENES PHYSIOLOGIQUEMENT ACTIFS

(30) Priorität: 30.09.1991 DE 4132587
(43) Veröffentlichungstag der Anmeldung: 15.09.1993
(73) Patentinhaber: B.R.A.H.M.S Diagnostica GmbH, D-12099 Berlin (DE)
(72) Erfinder: BERGMANN, Andreas, D-1000 Berlin 47 (DE)
(74) Vertreter: Andrae, Steffen, Dr.
(86) Internationale Anmeldenummer: EP9201855
(87) Internationale Veröffentlichungsnummer: WO9307489

(56) Entgegenhaltungen:
- EP-A- 0 306 167
- EP-A- 0 362 773
- EP-A- 0 385 488
- US-A- 5 015 627
- US-A- 5 039 446

## Beschreibung

Die Erfindung betrifft ein Verfahren zur Stabilisierung von endogenen, physiologisch aktiven Peptiden in humanen Vollblut-, Serums- oder Plasmaproben vor und/oder während der Bestimmung der Konzentration dieser Peptide nach immundiagnostischen oder physikalischen Bestimmungsverfahren.

Peptide haben wichtige biologische Funktionen als Hormone und Biomodulatoren. Aufgrund ihrer hohen biologischen Wirksamkeit ist ihre Halbwertszeit im Organismus stark begrenzt, und für die verschiedenen physiologisch aktiven Peptide existieren verschiedene, der biologischen Funktion dieser Peptide entsprechende Desaktivierungs- und Abbaumechanismen. Ein Überblick über eine Reihe von physiologisch wichtigen Peptiden bzw. Peptidhormonen und deren Desaktivierung im menschlichen Organismus findet sich beispielsweise in dem Aufsatz von Hugh P.J. Bennett und Colin McMartin in Pharmacological Reviews, Vol. 30, No. 3, Seiten 247 bis 292. Diesem Artikel ist zu entnehmen, daß die Halbwertszeit von Peptiden im Blut von einer Vielzahl verschiedener Faktoren beeinflußt wird, zu denen die Aufnahme bzw. Bindung durch das Gewebe, ein Abbau durch spezifische Gewebebereiche oder Organe sowie auch ein Abbau oder eine Umwandlung im Blut oder Plasma gehören.

Will man im Blut von Versuchstieren oder insbesondere von menschlichen Patienten die Konzentration derartiger kurzlebiger Peptide bestimmen, muß man diese Kurzlebigkeit in Rechnung stellen und gegebenenfalls Gegenmaßnahmen ergreifen.

Zur Bestimmung der Konzentration von Biomolekülen, wie Peptiden, im Blut muß zuerst eine Blutprobe genommen werden, aus der man dann auf bekannte Weise eine Serumsprobe oder Plasmaprobe gewinnt, die man dann in das Bestimmungsverfahren zur Konzentrationsbestimmung des jeweiligen Biomoleküls einsetzt. Obwohl die Konzentration verschiedener Biomoleküle auch mittels physikalischer Methoden, wie chromatographischer Methoden, spektroskopischer Methoden oder durch Abtrennung wie Dialyse oder Ultrafiltration, gemessen werden kann, werden Biomoleküle im Blut aufgrund der geringen Mengen, in denen sie vorkommen, und aufgrund der bei klinischen Untersuchungen erforderlichen hohen Bestimmungsgenauigkeit in der Regel mittels verschiedener immundiagnostischer Methoden gemessen. Bei diesen Methoden wird die Serumsprobe bzw. Plasmaprobe zusammen mit den weiteren, für das jeweilige immundiagnostische Verfahren erforderlichen Substanzen (markierte oder unmarkierte, suspendierte oder immobilisierte Antikörper, markierte Tracermoleküle, Puffer) für bestimmte Zeiten miteinander inkubiert.

Normalerweise vergeht zwischen der Probenentnahme und der Gewinnung von Serums- bzw. Plasmaproben und der eigentlichen Messung des Biomoleküls ein erheblicher Zeitraum. Im Falle empfindlicher endogener Peptide kann es jedoch im Zeitraum zwischen Probenentnahme bzw. Probeaufarbeitung und eigentlicher Bestimmung zu einem endogenen biologischen Abbau der Peptide in der Probe kommen, und auch während der Zeit der Inkubation der Probe während des Bestimmungsverfahrens kann sich der endogene biologische Abbau der Peptide fortsetzen, so daß, abhängig vom Grade des Abbaus, bei dem Bestimmungsverfahren nicht die gesuchte physiologische Peptidkonzentration, die zum Zeitpunkt der Blutentnahme vorhanden war, gemessen wird, sondern eine andere, deren Größe abhängig ist von der Art und Dauer der Lagerung, der Serums/Plasma-Gewinnung und der Inkubation. Wird bei dem Bestimmungsverfahren nur das vollständig, nicht abgebaute Peptid erkannt, werden somit zu niedrige Werte erhalten. Es gibt jedoch auch immundiagnostische Bestimmungsverfahren, in denen mögliche Abbauprodukte eines bestimmten Peptids besser erkannt werden als das Peptid selbst, so daß im Falle eines Abbaus des gesuchten Peptids auch höhere als die tatsächlichen Konzentrationen gemessen werden können.

Falsche Meßergebnisse führen jedoch gegebenenfalls zu falschen klinischen Interpretationen der Meßergebnisse.

Um der schon seit längerem grundsätzlich bekannten Gefahr des Abbaus endogener Peptide zu begegnen, ist der Versuch bekannt, einen derartigen Abbau durch Anwesenheit von EDTA oder auch von einzelnen Proteolysehemmstoffen zu verhindern. Ferner ist es bekannt, die Peptide durch sofortiges Einfrieren der Proben oder durch Lyophilisation der Proben zu stabilisieren. Es ist ferner auch bekannt, den Abbau der zu bestimmenden Peptide durch eine sogenannte Hitzebehandlung zu vermindern, wobei für einen proteolytischen Abbau verantwortliche Proteasen entaktiviert werden.

Obwohl grundsätzlich davon ausgegangen wird, daß am Abbau endogener Peptide ein proteolytischer Abbau beteiligt ist, ist es bisher in der Regel nicht bekannt, wie dieser Abbau im einzelnen erfolgt und wie er für ein spezifisches endogenes Peptid im Serum/Plasma wirksam unterdrückt werden kann.

Physiologisch aktive endogene Peptide, die nur eine begrenzte Lebensdauer aufweisen oder mögliche Kandidaten für einen proteolytischen Abbau in Blut-, Serums- oder Plasmaproben sind, sind beispielsweise das adrenokortikotrope Hormon (Corticotropin, ACTH), die Angiotensine, die atrialen Peptide, einschließlich des atrialen natriuretischen Peptids (ANP), Bradykinin, Calcitonin, Calcitonin-Vorläufer und das zum Calcitonin-Gen in Beziehung stehende Peptid (calcitonin gene-related peptide), Cholecystokinin, Glucagon, Interleukine, Insulin, Katacalcin (PDN-21), das luteinisierendes Hormon freisetzende Hormon (LHRH) und das Parathormon oder Parathyroidhormon (PTH).

Im Rahmen der vorliegenden Erfindung liegt dabei der Schwerpunkt auf der Stabilisierung der instabilen Peptide ACTH (adrenokortikotropes Hormom) und ANP (atriales natriuretisches Peptid).

Die Struktur dieser beiden Peptide ist bekannt und wird nachfolgend angegeben:

### Humanes ACTH (1-39)

Ser-Tyr-Ser-Met-Glu-His-Phe-Arg-Trp-Gly-Lys-Pro-Val-Gly-Lys-
Lys-Arg-Arg-Pro-Val-Lys-Val-Tyr-Pro-Asn-Gly-Ala-Glu-Asp-Glu-
Ser-Ala-Glu-Ala-Phe-Pro-Leu-Glu-Phe

### Humanes ANP (1-28)

Ser-Leu-Arg-Arg-Ser-Ser-Cys-Phe-Gly-Gly-Arg-Met-Asp-Arg-Ile-
Gly-Ala-Gin-Ser-Gly-Leu-Gly-Cys-Asn-Ser-Phe-Arg-Tyr

Die angegebenen Strukturen sind veröffentlicht in K. Kangawa und H. Matuso; Biochem. Biophys. Res. Commun. 118, 131 (1984) bzw. M. Marin-Grez et al., Life Sci., 36, 2171 (1985).

Die beiden Peptide hACTP und hANP sind sowohl was ihre Größe, ihre Struktur (ANP ist z.B. ein cyclisches Peptid) als auch was ihre Aminosäuresequenz angeht so stark voneinander verschieden, daß im Rahmen der vorliegenden Erfindung davon ausgegangen wird, daß ein Stabilisierungsverfahren, das für beide Peptide in Serumsproben bzw. Plasmaproben ähnlich wirksam ist, auch für die Stabilisierung aller oder zumindestens vieler weiterer endogener, physiologisch aktiver Peptide geeignet ist.

Der Erfindung liegt die Aufgabe zugrunde, ein Verfahren zu schaffen, durch das endogene, physiologisch aktive Peptide in humanen Vollblut- sowie insbesondere humanen Serums- und Plasmaproben so stabilisiert werden können, daß ihre Konzentration vor und während der Bestimmung nicht durch einen die Meßergebnisse verfälschenden Abbau verändert werden.

Diese Aufgabe wird durch ein Verfahren zur Stabilisierung von endogenen, physiologisch aktiven Peptiden in humanen Vollblut-, Serums- oder Plasmaproben vor und/oder während der Bestimmung der Konzentration dieser Peptide nach immundiagnostischen oder auch physikalischen Bestimmungsverfahren gelöst, das dadurch gekennzeichnet ist, daß man den Proben eine Stabilisatorenkombination zusetzt, die aus den Proteaseninhibitoren Amastatin und Leupeptin sowie Ethylendiamintetraessigsäure (EDTA) besteht oder die genannten Verbindungen enthält, z.B. in Form einer Lösung in einem geeigneten Puffer.

Es wurde dabei ferner festgestellt, daß normalerweise die Komponenten der Stabilisatorenkombination der jeweiligen Probe, insbesondere Serums- oder Plasmaprobe, in solchen Mengen zugesetzt werden müssen, daß in der jeweiligen Probe Mindestkonzentrationen von 4 mM EDTA, 25 µM Amastatin und 50 µM Leupeptin enthalten sind.

### Das erfindungsgemäße Verfahren wird in der vorliegenden

Erfindung insbesondere im Hinblick auf die Bestimmung von humanem ACTH in Serums- und Plasmaproben bzw. im Hinblick auf seine Verwirklichung mittels eines entsprechenden Kits noch näher erläutert.

Das erfindungsgemäße Verfahren hat im Hinblick darauf, daß eine Kombination von Leupeptin und Amastatin zur Peptidstabilisierung verwendet wird, eine gewisse Ähnlichkeit mit dem Verfahren der Anmelderin, das in derem deutschen Patent 38 33 936 beschrieben ist. Das in dem genannten Patent beschriebene Verfahren betrifft jedoch nicht die Stabilisierung irgendeines zu messenden endogenen Peptids, sondern die Stabilisierung eines bestimmten, künstlich hergestellten Oligopeptidtracers, der in einem typischen Immunoassay, bei dem die zu bestimmende Substanz und ein Tracer um eine unterschüssige Antikörpermenge miteinander konkurrieren, zugesetzt wird. Durch die Zugabe einer Kombination von Leupeptin und Amastatin kann ein die Meßergebnisse verfälschender Abbau dieses Oligopeptidtracers während der Durchführung der Bestimmung vermieden werden.

Dem genannten Patent können keine Hinweise entnommen werden, daß die zur Stabilisierung des Oligopeptidtracers verwendete Kombination von Leupeptin und Amastatin auch im Zusammenhang mit der Stabilisierung anderer Peptide geeignet sein könnte oder auch im Sinne einer Stabilisierung des im genannten Patent zu bestimmenden Peptids wirksam wäre. Es war vielmehr außerordentlich überraschend, als im Zusammenhang mit der Schaffung der vorliegenden Erfindung festgestellt wurde, daß eine Mischung von Leupeptin und Amastatin dann, wenn als dritte Komponente auch noch EDTA anwesend ist, endogene Peptide ganz verschiedener Natur in Blutproben und insbesondere in Serums- und Plasmaproben stabilisiert werden können und daß offensichtlich die genannte Stabilisatorenmischung im Sinne einer Ausschaltung der für den Abbau der meisten endogenen Peptide in derartigen Proben verantwortlichen Substanzen wirksam ist.

Nachfolgend wird die vorliegende Erfindung anhand von Beispielen noch näher erläutert.

### Beispiel 1

Zur Bestimmung des Abbaus der Peptide hACTH 1-39 und hANP 1-28 in humanem Serum bzw. humanem Plasma in Gegenwart verschiedener als Proteaseinhibitoren bekannter Substanzen wurden mit ¹²⁵I radioiodierte ACTH sowie entsprechend radio-iodiertes ¹²⁵I-ANP 1-28 mit Serum und Plasma inkubiert und somit dem Abbau durch endogene Proteasen ausgesetzt, die in humanem Serum und Plasma wirksam werden. Nach bestimmten Zeitintervallen wurden das Reaktionsgemisch jeweils mittels reversed-phase-HPLC chromatographisch analysiert, wobei das Ergebnis der Chromatographie kontinuierlich mit einem Radio-aktivitätsdetektor gemessen wurde. Die Ausgangssubstanzen (t = 0) werden als symmetrische radioaktive Peaks eluiert. Entstehende Abbauprodukte liefern u.a. weitere radioaktive Peaks. Der prozentuale Abbau der Ausgangssubstanzen ergibt sich aus dem Peakintegral des Produkts oder der Produkte geteilt durch die Summe der Peakintegrale von unverändertem Edukt und allen Produkten mal 100.

In einem Kontrollversuch wird der normale Abbau der Ausgangspeptide gemessen, und dann wird die Messung unter identischen Bedingungen in Anwesenheit unterschiedlicher potentieller Proteolysehemmstoffe wiederholt.

Zur Messung des Abbaus bzw. des Einflusses verschiedener potentieller Hemmstoffe wurde im einzelnen wie folgt vorgegangen:

¹²⁵I-ACTH oder ¹²⁵I-ANP (1,5 Mio cpm) in 20 µl Phosphatpuffer (250 mM, pH 7,4) wurden mit 5 µl der jeweilig zu testenden Inhibitorlösung in 15facher Konzentration gemischt (Kontrollen wurden mit 5 µl Wasser vermischt). Die Reaktion wurde dann durch Zugabe von 50 µl frisch gewonnenem Serum oder EDTA-Plasmna gestartet. Nach der Zugabe wurden die Ansätze jeweils bei 22°C inkubiert (ACTH in Serum für 18 h, ACTH in Plasma für 25 h, ANP in Serum für 1 h, ANP in Plasma für 3 h). Gestoppt wurden die Reaktionen durch Einfrieren der Proben. Unmittelbar vor der chromatographischen HPLC-Analyse wurden die Proben aufgetaut und mit 1 ml des nachfolgend erläuterten Laufmittels A versetzt, durch 0,2 pm Filter sterilfiltriert und mittels einer µ-Bondapack-C18-Säule (0,4 x 30 cm) der Firma Waters über HPLC aufgetrennt.

Die Elution der Substanzen erfolgte unter Verwendung eines Gradienten, der aus einem Laufmittel A (LMA) aus Acetonitril:Wasser:Trifluoressigsäure iom Volumenverhältnis 5:95:0,1 und einem Laufmittel B (LMB) aus Acetonitril:Wasser:Trifluoressigsäure im Volumenverhältnis 90:10:0,1 wie folgt erzeugt wurde:

In 45 min linear von 95/5 (V/V LMA/LMB) (Startbedingungen) nach 65/35 LMA/LMB. Die Durchflußgeschwindigkeit betrug 5 ml/min. Die Radioaktivität des Säuleneluats wurde kontinuierlich mittels eines Radioaktivitätsmonitors (Firma Raytest) verfolgt. Der Abbau der radioaktiven Peptide (in % Umsatz) wurde aus dem resultierenden Abbaumuster und unter Verwendung eines Computerprogramms (Raytest) ermittelt.

Es zeigte sich dabei, daß die in die Abbauversuche eingesetzten Peptide sich bei ihrer Untersuchung mittels HPLC von einer Vielzahl ihrer Fragmente abtrennen ließen, so daß der Abbau der Peptide leicht untersucht werden konnte. Ohne Zusatz von Proteolysehemmstoffen oder bei Zusatz ungeeigneter Proteolysehemmstoffe wird bei 22°C unter den angegebenen Bedingungen nach der entsprechenden Inkubation mit Serum oder Plasma ein 35 bis 70 %iger Abbau des eingesetzten markierten Peptids beobachtet.

Die in die Versuche eingesetzten Hemmstoffe (Inhibitoren) waren jeweils Handelsprodukte. Nachfolgend sind die zusammen mit ihrer jeweiligen Bezugsquelle (in Klammern) aufgelistet:

EDTA (Fluka 03610), DPFP (Serva 77205), PMSF (Merck 7349), CCPS (Sigma C-4503), NEM (Serva 11331), Bestatin (Novabiochem A02341), Amastatin (Biomol 50360), Pepstatin (Serva 52682), Elastatinal (Sigma E-0881), Leupeptin (Biomol 12136), Phosphoramidon (Novabiochem A01239), Benzamidin (Sigma B-6505), Trasylol bzw. Aprotinin (Sigma A-6012), Heparin (Serva 63036), Sojabohnen-Trypsin-Inhibitor (Sigma T-900), Antithrombin III (Sigma A-7388).

Die erhaltenen Ergebnisse sind in der folgenden Tabelle 1 zusammengefaßt.

Die untersuchten Peptide wurden bezogen von: hACTH (Bachem PACT100), hANP (Novabiochem 05-23-0300).

**Tabelle 1**

| Einfluß verschiedener Protease-Inhibitoren auf die Hydrolyse von ¹²⁵I-ACTH 1-39 | | | |
|---|---|---|---|
| Hemmstoff | Konzentration | % der Hydrolysegeschwindigkeit der Kontrolle | |
| | | in Serum | in EDTA-Plasma |
| Kontrolle | / | 100 | 100 |
| Ethylendiamintetraacetat | 10 mM | 30 | / |
| Diisopropylfluorphosphat | 1 mM | 83 | 95 |
| Phenylmethylsulfonylfluorid | 2 mM | 97 | 100 |
| p-Chloromercuryphenylsulfonsäure | 1 mM | 94 | 62 |
| N-Ethylmaleimid | 2 mM | 48 | 54 |
| Bestatin | 1 mM | 44 | 27 |
| Amastatin | 100 µM | 17 | 3 |
| Pepstatin | 100 µM | 105 | 78 |
| Elastatinal | 100 µM | 97 | 100 |
| Leupeptin | 1 mM | 41 | 27 |
| Phosphoramidon | 1 mM | 98 | 85 |
| Benzamidin | 1 mM | 84 | 80 |
| Trasylol | 2,5 Einheiten/ml | 78 | 62 |
| Heparin | 5 mg/ml | 126 | 103 |
| Trypsin-Inhibitor (Soybean) | 0,1 mg/ml | 78 | 71 |
| Antithrombin III | 0,1 Einheiten/ml | 107 | 79 |
| Leupeptin | 1 mM + | | |
| Amastatin | 100 µM | 14 | 0 |
| Leupeptin | 1 mM + | | |
| Amastatin | 100 µM + | | |
| EDTA | 10 mM | 0 | 0 |

Der geschilderten Untersuchung unter Einsatz von ¹²⁵I-ACTH 1-39 ist zu entnehmen, daß eine Kombination von Leupeptin/ Amastatin/EDTA die Hydrolyse im Serum vollständig unterdrückte, während in einem bereits EDTA enthaltenden Plasma der gleiche Effekt durch Zugabe von Leupeptin und Amastatin allein erzielt wurde.

Der Tabelle ist ferner zu entnehmen, daß bemerkenswerterweise Hemmstoffe, die dem gleichen Inhibitorentyp angehören, also beispielsweise wie Amastatin Aminopeptidasenhibitoren darstellen, unwirksam oder sehr viel weniger wirksam sind.

Im Hinblick auf die für ¹²⁵I-ACTH erhaltenen Ergebnisse wurde der Einfluß der wirksamsten Hemmstoffkonzentration auch im Hinblick auf den Abbau von ¹²⁵I-ANP 1-28 untersucht. Die Ergebnisse sind in der nachfolgenden Tabelle 2 zusammengefaßt.

**Tabelle 2**

| Einfluß von Protease-Inhibitoren auf die Hydrolyse von ¹²⁵I-ANP 1-28 | | | |
|---|---|---|---|
| Hemmstoff | Konzentration | % der Hydrolysegeschwindigkeit der Kontrolle | |
| | | in Serum | in EDTA-Plasma |
| Kontrolle | / | 100 | 100 |
| | | | |
| Leupeptin | 1 mM + | | |
| Amastatin | 100 µM + | / | < 10 |
| | | | |
| Leupeptin | 1 mM + | | |
| Amastatin | 100 µM + | | |
| EDTA | 10 mM | < 10 | < 10 |

Tabelle 2 ist zu entnehmen, daß eine Mischung an Amastatin, Leupeptin und EDTA nicht nur eine Stabilisierung von ACTH im Serum bzw. Plasma bewirkt, sondern auch das ganz anders aufgebaute cyclische Peptid ANP ähnlich wirksam stabilisiert.

Zur Ermittlung der zu einem ausreichenden Schutz der Peptide erforderlichen Konzentrationen der einzelnen Inhibitoren in Anwesenheit der anderen Inhibitoren wurden die Konzentrationen mehrfach variiert. Es ergab sich, daß für einen wirksamen Schutz die in der Probe vorliegenden Mindestkonzentrationen für EDTA etwa 4 mM, an Amastatin etwa 25 µM und an Leupeptin 50 µM betragen.

### Praktisches Anwendungsbeispiel (hACTH-Kits)

Nachfolgend wird die praktische Nutzung des erfindungsgemäßen Verfahrens im Rahmen von immunometrischen Assays zur ACTH-Bestimmung der Anmelderin beispielhaft erläutert.

Ein Kit zur Durchführung eines immunoradiometrischen Bestimmungsverfahrens für ACTH (DYNo^{R}test ACTH von Henning Berlin, für die Markteinführung vorbereitet) enthält folgende, für einen Kit der vorliegenden Art typische Bestandteile:
a. Den Tracer in Form von ¹²⁵I-Anti-ACTH (34-39)-Antikörper (Maus, monoklonal), eine Flasche à 10 ml, rot eingefärbt, Konzentrat, das vor Gebrauch mit 23 ml Tracerpuffer zu rekonstituieren ist.
b. Den Tracerrekonstitutionspuffer, eine Flasche mit 23 ml, gebrauchsfertig.
c. Coated tubes (Teströhrchen), beschichtet mit immobilisiertem Anti-ACTH (25-21)-Antikörper (Maus, monoklonal), zweimal 50 Stück, gebrauchsfertig.
d. Waschlösung, 2 Flaschen à 11 ml, Konzentrat. Vor Gebrauch ist der Inhalt jeder Flasche mit destilliertem Wasser auf ein Endvolumen von 550 ml aufzufüllen.
e. ACTH-Nullstandard (Humanserum), 1 Flasche, à 10 ml gebrauchsfertig.
f. 1 bis 6 ACTH-Standards, intaktes humanes ACTH (1-39), in 6 Fläschchen, lyophilisiert. Die Konzentrationen betragen 5; 15; 50; 150; 500; 1500 pg/ml. Vor Gebrauch sind die Standards mit je 1 ml Nullserum zu rekonstituieren.
i,ii. Kontrollseren I und II (Humanserum), 2 Flaschen, lyophilisiert.

Außerdem enthält der Kit noch einen Puffer A, der die Proteaseninhibitoren zur Hemmung des ACTH-Abbaus in lyophilisierter Form enthält und vor Gebrauch in 11 ml eines ebenfalls beigefügten Puffers B zu lösen ist.

Zur Verhinderung des Abbaus von ACTH vor bzw. während der eigentlichen Bestimmung wird als erster Schritt des Verfahrens die durch Mischung des lyophilisierten Puffers A mit dem flüssigen Puffer B erhaltene Lösung in die jeweiligen Teströhrchen für die Standards und die Patentientenseren überführt, d.h. der stabilisierende Puffer wird vorgelegt, und die ACTH-haltigen Bestandteile werden während des Tests von Anfang an durch den Puffer A stabilisiert.

Die Messung erfolgt dann auf an sich bekannte Weise dadurch, daß man die Seren bzw. Standards in den beschichteten Teströhrchen inkubiert, anschließend eine Waschlösung zugibt und eine Fest/Flüssig-Trennung durchführt und diesen Schritt gegebenenfalls wiederholt, wonach zur Markierung der an die feste Phase gebundenen ACTH-Moleküle der markierte Antikörper in einem geeigneten Puffer in einem weiteren Testschritt zugegeben wird. Nach der erfolgten Inkubation, einer Fest/Flüssig-Trennung und dem Waschen wird die gebundene Radioaktivität gemessen und daraus die hACTH-Konzentration in der jeweiligen Probe unter Berücksichtigung der für den Standard erhaltenen Werte ermittelt.

Anstelle des radioaktiv markierten Tracer-Antikörpers bei dem oben beschriebenen immunoradiometrischen Assay wird bei einem ansonsten weitgehend ähnlichen immunoluminetrischen Assay in dem entsprechenden Kit (LUMI^{R}test ACTH von Henning Berlin, für die Markteinführung vorbereitet) ein lumineszenzmarkierter (beispielsweise mit einem Acridiniumderivat markierter) Anti-hACTH-(34-39)-Antikörper (monoklonal, Maus) verwendet. Auch in diesem Falle wird die die Inhibitoren enthaltende Pufferlösung in den Teströhrchen vorgelegt, bevor eine Patientenprobe oder ein Standard zugegeben wird.

Aufgrund der üblichen Praxis wird dabei in den obigen Fällen gefordert, daß die Patientenproben, die nicht innerhalb einer halben Stunde sofort in die Bestimmungen eingesetzt werden können, eingefroren werden und bei mindestens -20°C gelagert werden und nach dem Auftauen sofort weiterverarbeitet werden.

Es ist jedoch grundsätzlich auch möglich, die Patientenproben sofort nach ihrer Gewinnung durch Zusatz einer ausreichenden Mene der erfindungsgemäßen Stabilisatorenkombination zu stabilisieren. Zu diesem Zweck kann dem jeweiligen die Probe entnehmenden Arzt ein besonderer Kit zur Verfügung gestellt werden, der alle für die Probenstabilisierung erforderlichen Substanzen enthält.

## Patentansprüche

1. Verfahren zur Stabilisierung von endogenen, physiologisch aktiven Peptiden in humanen Vollblut-, Serums- oder Plasmaproben vor und/oder während der Bestimmung der Konzentration dieser Peptide nach immundiagnostischen oder physikalischen Bestimmungsverfahren, **dadurch gekennzeichnet,** daß man den Proben eine Stabilisatorenkombination zusetzt, die aus den Proteaseninhibitoren Amastatin und Leupeptin sowie Ethylendiamintetraessigsäure (EDTA) besteht oder die genannten Verbindungen enthält.

2. Verfahren nach Anspruch 1, dadurch gekennzeichnet, daß man die Stabilisierkombination einer Blutprobe sofort nach ihrer Entnahme aus einem Patienten zusetzt und die Gewinnung einer Serumsprobe und/oder Plasmaprobe in Gegenwart der genannten Stabilisatorenkombination durchführt.

3. Verfahren nach Anspruch 1, dadurch gekennzeichnet, daß man die Stabilisatorenkombination einer Blutprobe bzw. Serums- oder Plasmaprobe zusetzt, die durch Auftauen sowie gegebenenfalls sofortige anschließende Aufarbeitung unter Gewinnung einer Serums- oder Plasmaprobe ausgehend von einer sofort nach der Probennahme eingefrorenen Patientenblutprobe erhalten wurde.

4. Verfahren nach Anspruch 1, 2 oder 3, dadurch gekennzeichnet, daß man die Konzentrationen der Peptidhormone in der Serums- oder Plasmaprobe nach immundiagnostischen Bestimmungsverfahren bestimmt und die Bestimmung in Gegenwart der genannten Stabilisatorenkombination durchführt.

5. Verfahren nach einem der Ansprüche 1 bis 4, dadurch gekennzeichnet, daß das zu stabilisierende endogene Peptid ausgewählt ist aus einer Gruppe, die das adrenokortikotrope Hormon (Corticotropin, ACTH), die Angiotensine, die atrialen Peptide, einschließlich des atrialen natriuretischen Peptids (ANP), Bradykinin, Calcitonin, Calcitonin-Vorläufer und das mit dem Calcitonin-Gen in Beziehung stehende Peptid (calcitonin gene-related peptide), Cholecystokinin, Glucagon, Interleukine, Insulin, Katacalcin (PDN-21), das luteinisierendes Hormon freisetzende Hormon (LHRH) und das Parathormon (PTH) umfaßt.

6. Verfahren nach einem der Ansprüche 1 bis 5, dadurch gekennzeichnet, daß die Komponenten der Stabilisatorenkombination der jeweiligen Probe in solchen Mengen zugesetzt werden, daß in der jeweiligen Probe Mindestkonzentrationen von 4 mM EDTA, 25 µM Amastatin und 50 µM Leupeptin erhalten werden.

7. Verfahren nach einem der Ansprüche 1 bis 6, dadurch gekennzeichnet, daß es zur Stabilsierung vor und/oder während der Bestimmung von ACTH in Patientenproben, in denen ACTH nach einem mit markierten anti-ACTH-Antikörpern arbeitenden immunometrischen Assay bestimmt wird, eingesetzt wird.

8. Kit zur Stabilisierung von physiologisch wirksamen Peptiden in frischen oder in aufgetauten, sofort nach ihrer Gewinnung eingefrorenen Patienten-Blutproben oder daraus gewonnenen Serums- oder Plasmaproben, dadurch gekennzeichnet, daß er eine oder mehrere Lösungen enthält, in der bzw. denen EDTA, Amastatin und Leupeptin in den zur Probenstabilisierung erforderlichen Mengen vorliegen.

9. Kit zur immundiagnostischen Bestimmung von physiologisch aktiven Peptiden in Serums- oder Plasmaproben dadurch gekennzeichnet, daß er neben den eigentlichen Reagenzien für die immundiagnostische Bestimmung eine oder mehrere Lösung(en), in der bzw. denen EDTA, Amastatin und Leupeptin in zur Probenstabilisierung geeigneten Konzentrationen vorliegen sowie ggf. mit dieser Stabilisatorenkombination stabilisierte Standardlösungen der zu bestimmenden Substanz enthält.

10. Kit nach Anspruch 9, dadurch gekennzeichnet, daß er ein Kit zur immunometrischen Bestimmung von ACTH ist.

11. Kit nach Anspruch 10, dadurch gekennzeichnet, daß der Kit zur immunometrischen Bestimung von ACTH ein Kit für ein nach dem Sandwich-Prinzip arbeitendes immunometrisches Bestimmungsverfahren mit einem immobilisierten anti-ACTH-Antikörper und einem mit einem radioaktiven Tracermolekül oder einem Chemilumineszenzchromophor markierten zweiten anti-ACTH-Antikörper ist und daß er die Tracerkombination in Form einer Lösung in einem geeigneten Puffer enthält, die bei Testdurchführung vor der Zugabe der restlichen Testkomponenten in den Test- bzw. Standardröhrchen vorzulegen ist.

## Claims

1. Method for the stabilisation of endogenous, physiologically active peptides in human whole blood, serum or plasma samples before and/or during the determination of the concentration of these peptides by immunodiagnostic or physical assay methods, characterised in that a stabiliser combination which consists of the protease inhibitors amastatin and leupeptin and ethylenediaminetetraacetic acid (EDTA) or contains the stated compounds is added to the samples.

2. Method according to Claim 1, characterised in that the stabiliser combination is added to a blood sample immediately after its withdrawal from a patient, and a serum sample and/or plasma sample is isolated in the presence of the stated stabiliser combination.

3. Method according to Claim 1, characterised in that the stabiliser combination is added to a blood sample or serum or plasma sample which was obtained by thawing and, if necessary, immediate subsequent working-up with isolation of a serum or plasma sample starting from a patient's blood sample frozen immediately after being taken.

4. Method according to Claim 1, 2 or 3, characterised in that the concentrations of the peptide hormones in the serum or plasma sample are determined by immunodiagnostic assay methods, and the determination is carried out in the presence of the stated stabiliser combination.

5. Method according to any of Claims 1 to 4, characterised in that the endogenous peptide to be stabilised is selected from a group which comprises the adrenocorticotropic hormone (corticotropin, ACTH), the angiotensins, the atrial peptides, including atrial natriuretic peptide (ANP), bradykinin, calcitonin, calcitonin precursors and calcitonin gene-related peptide, cholecystokinin, glucagon, interleukins, insulin, katacalcin (PDN-21), luteinising hormone releasing hormone (LHRH) and parathormone (PTH).

6. Method according to any of Claims 1 to 5, characterised in that the components of the stabiliser combination are added to the particular sample in amounts such that minimum concentrations of 4 mM EDTA, 25 µM amastatin and 50 µM leupeptin are obtained in the particular sample.

7. Method according to any of Claims 1 to 6, characterised in that it is used for stabilisation before and/or during the determination of ACTH in patient samples in which ACTH is determined by an immunometric assay employing labelled anti-ACTH antibodies.

8. Kit for the stabilisation of physiologically active peptides in fresh or in thawed patient blood samples frozen immediately after being taken or serum or plasma samples obtained therefrom, characterised in that it contains one or more solutions in which EDTA, amastatin and leupeptin are present in the amounts required for sample stabilisation.

9. Kit for the immunodiagnostic determination of physiologically active peptides in serum or plasma samples, characterised in that, in addition to the actual reagents for the immunodiagnostic determination, it contains one or more solution(s) in which EDTA, amastatin and leupeptin are present in concentrations suitable for sample stabilisation, and standard solutions of the substance to be determined which, if required, are stabilised with the stabiliser combination.

10. Kit according to Claim 9, characterised in that it is a kit for the immunometric determination of ACTH.

11. Kit according to Claim 10, characterised in that the kit for the immunometric determination of ACTH is a kit for an immunometric assay method based on the sandwich principle, with an immobilised anti-ACTH antibody and a second anti-ACTH antibody labelled with a radioactive tracer molecule or a chemiluminescent chromophore, and that it contains the tracer combination in the form of a solution in a suitable buffer, which solution must be taken in the test tube or standard tube before the addition of the remaining test components when the test is carried out.

## Revendications

1. Procédé pour la stabilisation de peptides endogènes, physiologiquement actifs, dans des échantillons de sang entier, de sérum ou de plasma humain, avant et/ou pendant la détermination de la concentration de ces peptides par des procédés immunologiques ou physiques de détermination, caractérisé en ce que l'on ajoute aux échantillons une combinaison de stabilisants qui est constituée des inhibiteurs de protéases amastatine et leupeptine ainsi que d'acide éthylènediaminetétraacétique (EDTA), ou qui contient lesdits composés.

2. Procédé selon la revendication 1, caractérisé en ce que l'on ajoute la combinaison de stabilisants à un échantillon de sang immédiatement après son prélèvement sur un patient, et que l'on réalise l'extraction d'un échantillon de sérum et/ou d'un échantillon de plasma en présence de ladite combinaison de stabilisants.

3. Procédé selon la revendication 1, caractérisé en ce que l'on ajoute la combinaison de stabilisants à un échantillon de sang ou à un échantillon de sérum ou de plasma, qui a été obtenu en dégelant et en traitant éventuellement ensuite et immédiatement, par extraction d'un échantillon de sérum ou de plasma, un échantillon de sang du patient congelé immédiatement après le prélèvement de l'échantillon.

4. Procédé selon la revendication 1, 2 ou 3, caractérisé en ce que l'on détermine la concentration de l'hormone peptidique dans l'échantillon de sérum ou de plasma par un procédé de détermination immunologique, et en ce que l'on effectue la détermination en présence de ladite combinaison de stabilisants.

5. Procédé selon l'une des revendications 1 à 4, caractérisé en ce que le peptide endogène à stabiliser est choisi dans un groupe qui comprend l'hormone adrénocorticotrope (corticotropine, ACTH), l'angiotensine, les atriopeptides, y compris l'atriopeptide natriurétique (ANP), la bradykinine, la calcitonine, des précurseurs de la calcitonine et le peptide associé au gène de la calcitonine ("calcitonin gene-related peptide), la cholécystokinine, le glucagon, l'interleukine, l'insuline, la catacalcine (PDN-21), l'hormone libérant l'hormone lutéo-stimulante (LHRH) et la parathormone (PTH).

6. Procédé selon l'une des revendications 1 à 5, caractérisé en ce que les composants de la combinaison de stabilisants sont ajoutés à chaque échantillon en quantités telles que, dans chaque échantillon, des concentrations minimales de 4 mM en EDTA, de 25 µM en amastatine et de 50 µM en leupeptine sont atteintes.

7. Procédé selon l'une des revendications 1 à 6, caractérisé en ce qu'il est utilisé pour la stabilisation, avant ou pendant la détermination de l'ACTH dans des échantillons de patient, dans lesquels l'ACTH est déterminé par une détermination immunométrique qui travaille avec des anticorps anti-ACTH marqués.

8. Trousse pour la stabilisation de peptides physiologiquement actifs dans des échantillons de sang de patient ou dans des échantillons de sérum ou de plasma obtenus à partir de ces derniers, frais ou décongelés, congelés immédiatement après leur obtention, caractérisée en ce qu'elle contient une ou plusieurs solutions dans lesquelles l'EDTA, l'amastatine et la leupeptine sont présentes dans les quantités nécessaires pour la stabilisation des échantillons.

9. Trousse pour la détermination immunologique de peptides physiologiquement actifs dans des échantillons de sérum ou de plasma, caractérisée en ce qu'en plus des réactifs proprement dits pour la détermination immunologique, elle contient une ou plusieurs solutions dans lesquelles l'EDTA, l'amastatine et la leupeptine sont présentes en concentrations convenant pour la stabilisation des échantillons, ainsi que des solutions standard de la substance à déterminer, éventuellement stabilisées avec cette combinaison de stabilisants.

10. Trousse selon la revendication 9, caractérisée en ce qu'elle est une trousse pour la détermination immunométrique de l'ACTH.

11. Trousse selon la revendication 10, caractérisé en ce que la trousse pour la détermination immunométrique de l'ACTH est une trousse pour un procédé de détermination immunométrique qui travaille suivant le principe à sandwich, avec un anticorps anti-ACTH immobilisé et un second anticorps anti-ACTH marqué avec une molécule-traceur radioactive ou un chromophore de chimioluminescence, et en ce qu'elle contient la combinaison de traceurs sous la forme d'une solution dans un tampon approprié, qui lors de l'exécution du test doit être ajoutée dans les tubes d'essai ou les tubes standard, avant l'addition des autres composants du test.
